# EUROPEAN PATENT APPLICATION

(11) **EP 1 656 929 A1**
(43) Date of publication of application: **17.05.2006**
(21) Application number: 06001811.6
(22) Date of filing: 24.10.2001
(51) Int. Cl.: A61K 8/97, A61Q 19/00, A61Q 19/02, A61Q 19/08

(54) **Skin treatment composition and method based on the use of Chromolaena Odorata**

(30) Priority: 02.11.2000 GB 0026828
(62) Divisional of application: 01992561.9
(71) Applicant: UNILEVER PLC, London EC4P 4BQ (GB); UNILEVER N.V., 3013 AL Rotterdam (NL)
(72) Inventor: Donovan, Robert Mark, 95160 Montmorency (FR)
(74) Representative: Acham, Nicholas Clive

(57) **Abstract**

A cosmetic method of providing at least one skin care benefit selected from treating or preventing wrinkling, sagging, aged and/or photodamaged skin; boosting decorin production in skin, boosting transglutaminase type I and type III activity in skin, increasing stratum corneum flexibility, improving skin desquamation and epidermal differentiation, lightening skin colour, controlling sebum secretion, soothing irritated, red and/or sensitive skin, improving skin texture, smoothness, firmness and generally increasing the quality of skin comprising an effective amount of an extract Chromolaena Odorata.

## Description

This invention relates to a cosmetic method of improving the condition and appearance of skin, and to the use of certain plant extracts in the preparation of topical compositions for improving the condition and appearance of skin. It particularly relates to the use of extracts of Chromolaena Odorata (also known for example as Siam Weed or Devils Weed) for the treatment of wrinkles and/or photodamaged skin. It also relates to topical compositions containing such extracts.

Skin is subject to deterioration through dermatological disorders, environmental abuse (wind, air conditioning, central heating) or through the normal ageing process (chronoageing) which may be accelerated by exposure of skin to sun (photoageing). In recent years the demand for cosmetic methods for improving the appearance and condition and, in particular, for reversing, reducing or preventing the visible signs of wrinkled, aged and/or photodamaged skin has grown enormously.

Consumers are increasingly seeking "anti-ageing" cosmetic products that reverse, treat or delay the visible signs of chronoageing and photoageing skin such as wrinkles, lines, sagging, hyperpigmentation and age spots.

Collagen, the predominant matrix skin protein is known to impart tensile strength to skin. It is also known in the art that the levels of collagen in skin are significantly reduced with aged and/or photodamaged skin. Many studies have shown that the levels of collagen type I in skin is decreased with age and/or with increased photodamage, (see for example Lavker, R. J. Inv.Derm., (1979), 73,79-66; Griffiths et al. N. Eng. J. med. (1993) 329, 530-535). The reduction of the levels of collagen in skin is accordingly associated with a decrease in the tensile strength of the skin, causing wrinkles and laxity. It is widely accepted that strengthening of the dermal matrix by boosting the levels of collagen in skin provides anti-ageing and dermal repair benefits. It is also known, for example from WO 99/47110, that decorin can act as a marker of skin repair.

Siam weed extract comprising extract of Chromolaena Odorata is a known skin treatment extract, which has found use in the treatment of soft tissue wounds, burns and skin infections, for example in Vietnam. Typical of prior art demonstrating this is "The Journal of Alternative and Complimentary Medicine", Vol. 2, No. 3, 1996 pp 335-343, Phan et al. This describes the use of the aqueous extract of Siam weed leaves for the above mentioned purposes, and the effect of Siam weed extract (termed as Eupolin by Phan et al.) on hydrated collagen lattice contraction by human dermal fibroblasts. Previously prepared petrolatum based Siam weed ointments have been found to contain flavonoids (salvigenin, sakuranetin, isosakuranetin, kaemferid, betulenol, 2-5-7-3-tetra-O-methylquercatagetin, tamarixetin, chalcone 1 and chalcane 2), essential oils (geyren, bornyl acetate, beta eubeden), saponin triterpenoids, tannins, organic acids, and trace elements. In vitro levels of Siam weed extract in topically used compositions were typically 50-200 µg/ml.

In addition, in "Plastic and Reconstructive Surgery", March 1998, "Enhanced proliferation of Fibroplasts and Endothelial Cells Treated with an Extract of the Leaves of Chromolaena Odorata (Eupolin), a Herbal Remedy for Treating Wounds", Phan et al., there is described the use of Siam weed extract to increase fibroblast and endothelial cell growth, again in the context of burns treatment. In particular, it was found that where skin grafts are used, the use of Siam weed extract speeds up the formation of granulation tissue, as well as increasing the rate of collagen synthesis. In this study Siam weed extract ointment was used at topical concentrations of 10-230 µg/ml.

We have suprisingly found that effective treatment and prevention of normal skin conditions due to chronoageing or photoageing, such as wrinkles, lines, sagging, hyperpigmentation and age spots may be obtained through the topical application of cosmetic compositions containing extracts of Chromolaena Odorata. We have also found that the use of extracts of Chromolaena Odorata in cosmetic compositions may provide further benefits in addition to anti-ageing, such as for example soothing sensitive and/or irritated skin.

Thus according to a first aspect, there is provided a cosmetic method of providing at least one skin care benefit selected from treating or preventing wrinkling, sagging, aged and/or photodamaged skin; boosting decorin production in skin, boosting transglutaminase type I and type III activity in skin, increasing stratum corneum flexibility, improving skin desquamation and epidermal differentiation, lightening skin colour, controlling sebum secretion, soothing irritated, red and/or sensitive skin, improving skin texture, smoothness, firmness and generally increasing the quality of skin; the method comprising applying to the skin a topical composition comprising preferably an aqueous extract of Chromolaena Odorata.

According to a further aspect, there is provided in the use in the manufacture of a composition for the treatment inter alia of photodamaged skin and fine lines and wrinkles of an effective amount of an aqueous extract of Chromolaena Odorata.

According to yet a further aspect, there is provided a topical composition, preferably a water based topical composition for application to the human skin, comprising an effective amount of an aqueous extract of Chromolaena Odorata.

Preferably, topical compositions for use in the method according to the invention comprise extract of Chromolaena Odorata at a level of 0.00005 to 10 % of the topical composition, more preferably levels of 0.0001 to 1.0%, and in certain circumstances most preferably 0.001 to 0.1% by weight of the topical composition.

By "water based" is meant that the topical compositions preferably comprises at least 30%, more preferably at 60% by weight of the topical compositions of water. Conveniently, the topical product may be in the form of an oil-in-water emulsion.

In relation to the repair of photodamaged skin, it is generally accepted that the biochemical and biological processes associated with wound repair and remodelling of the dermis are not the same as those involved in photodamage repair. For example, other materials such as marigold and myrrh extracts, which induce wound repair in skin, are known not to be effective at photodamage repair.

The inventive compositions, methods and uses thus provide anti-ageing benefits which may result in the promotion of smooth and supple skin with improved elasticity and a reduced or delayed appearance of wrinkles and aged skin, with improved skin colour. A general improvement in the appearance, texture and condition, in particular with respect to the radiance, clarity, and general youthful appearance of skin may also be achieved. The invention may have particular utility for providing skin care benefits.

The term "treating" as used herein includes within its scope reducing, delaying and/or preventing the above mentioned normal but cosmetically undesirable skin conditions caused by the normal aging process. The visible signs of ageing such as wrinkles, lines and/or sagging may be delayed or reduced. Generally, the quality of skin may be enhanced and its appearance and texture may be improved by preventing or reducing wrinkling and increasing flexibility, firmness, smoothness, suppleness and elasticity of the skin.

The compositions, methods and uses according to the invention may be useful for treating skin that is already in a wrinkled, aged, and/or photodamaged condition, or for treating youthful skin to prevent or reduce those aforementioned undesirable changes due to the normal ageing/photoageing process.

The composition used according to the invention also comprises a dermatologically/cosmetically acceptable vehicle to act as a dilutant, dispersant or carrier for the actives. The vehicle may comprise materials commonly employed in skin care products such as water, liquid or solid emollients, silicone oils, emulsifiers, solvents, humectants, thickeners, powders, propellants and the like.

The vehicle will typically form from 5% to 99.9%, preferably from 25% to 80% by weight of the composition, and can, in the absence of other cosmetic adjuncts, form the balance of the composition.

Besides the extract of Chromolaena Odorata active, other specific skin-benefits actives such as sunscreens, skin-lightening agents, and skin tanning agents may also be included. The vehicle may also further include adjuncts such as antioxidants, perfumes, opacifiers, preservatives, colourants and buffers.

To prepare the topical composition used in the method of the present invention, the usual manner for preparing skin care products may be employed. The active components are generally incorporated in a dermatologically/cosmetically acceptable carrier in conventional manner. The active components can suitably first be dissolved or dispersed in a portion of the water or another solvent or liquid to be incorporated in the composition. The preferred compositions are oil-in-water or water-in-oil or water-in-oil-in-water emulsions, though under certain circumstances water in oil compositions are preferred.

The composition may be in the form of conventional skin-care products such as a cream, gel or lotion, capsules or the like. The composition can also be in the form of a so-called "wash-off" product e.g. a bath or shower gel, possibly containing a delivery system for the actives to promote adherence to the skin during rinsing. Most preferably the product is a "leave-on" product, that is a product to be applied to the skin without a deliberate rinsing step soon after its application to the skin.

The composition may be packaged in any suitable manner such as in a jar, a bottle, tube, roll-ball, or the like, in the conventional manner. A further envisaged form is as a formulation suitable for delivery as a spray, either from a propellant driven aerosol or from a pump spray.The composition according to the invention may also be formulated into a form suitable for oral ingestion such as a capsule, tablet or similar.

The method of the present invention may be carried out one or more times daily to the skin which requires treatment. The improvement in skin appearance will usually become visible after 3 to 6 months or possible sooner, depending on skin condition, the concentration of the active components used in the inventive method, the amount of composition used and the frequency with which it is applied. In general, a small quantity of the composition, for example from 0.1 to 5 ml is applied to the skin from a suitable container or applicator and spread over and/or rubbed into the skin using the hands or fingers or a suitable device. A rinsing step may optionally follow, depending on whether the composition is formulated as a "leave-on" or a "rinse-off' product.

In order that the present invention may be more readily understood, the following examples are provided by way of illustration only, with reference to the accompanying figure in which:

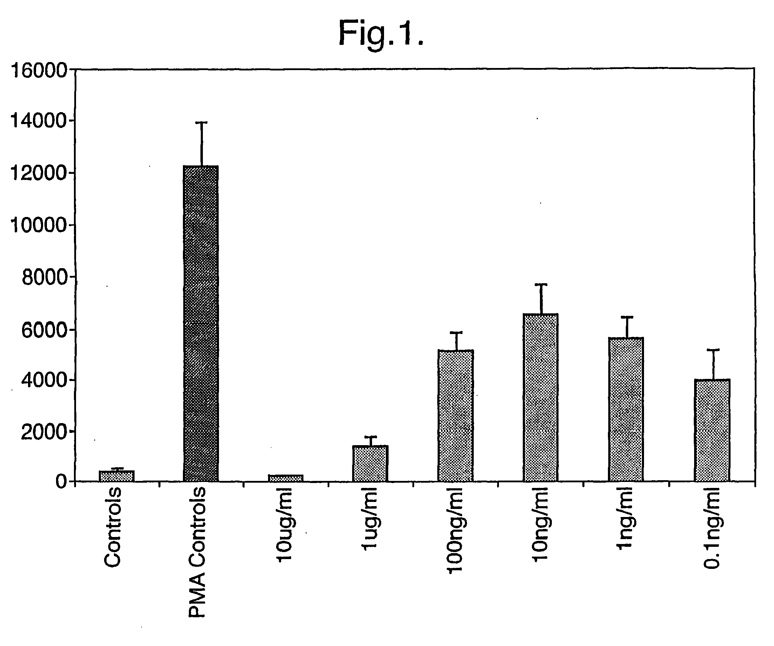

- Figure 1 shows the effect of Eupolin on PMA stimulated PGE2 levels in human skin fibroblasts.

### EXAMPLES

It is known from our co-pending European application no. 99908956.8 that topical retinoic acid treatments can be used to cause upregulation of procollagen I and decorin in vivo. To this end, the passages under the heading "Identification of procollagen I and decorin upregulation in skin in vivo following topical retinoic acid treatment for comparative purposes" in that application are incorporated herein in their entirety.

### Example 1

### Procedure For Measuring Procollagen-I and Decorin Synthesis In Human Dermal Fibroblasts

### Preparation of Dermal Fibroblast Conditioned Medium.

Primary human foreskin fibroblasts at passage 2 (P2) were seeded into 12-well plates at 10000 cells/cm2 and maintained for 24 hours in an atmosphere of 5% carbon dioxide and 4% oxygen in Dulbeccos Modified Eagles Medium (DMEM) supplemented with 10% foetal calf serum. After this time the cells were washed with serum free DMEM and then incubated in fresh serum free DMEM for a further 60 hours. The fibroblast monolayers were then washed again with serum free DMEM. Test reagents and vehicle controls were added to the cells in triplicate in a final volume of 0.4ml/well fresh serum free DMEM and incubated for a further 24 hours. This fibroblast conditioned medium was either analysed immediately or snap frozen in liquid nitrogen and stored at -70oC for future analysis. The cells were then counted and data from the dot-blot analysis subsequently standardised to cell number.

### Dot Blot Assay for Procollagen-I and Decorin Protein in Dermal Fibroblast Conditioned Medium

Samples of conditioned medium from dermal fibroblasts treated with vehicle (as a control) or test reagents were supplement with 20mM dithiothreitol (1:10 dilution of 200mM stock solution) and 0.1% sodium dodecylsuphate (1:100 dilution of 10% stock solution), mixed well and then incubated at 75oC for 2 minutes. A standard for the assay was generated by serial dilution of neat fibroblast conditioned medium from fibroblasts seeded at 10000 cells/cm2 in a 175cm2 flask and maintained in serum free DMEM as described above.

Assay samples were subsequently applied in triplicate to a pre-wetted sheet of Immobilon-P transfer membrane using the 96-well Bio-Dot Apparatus from Bio-Rad as described in the manufacturers guidelines. Approximately 200µl of medium was applied per well. The medium was allowed to filter through the membrane under gravity (30 minutes) after which the membrane was washed twice with PBS (200µl). These PBS washes were allowed to filter through the membrane under gravity (2x15 minutes). The Bio-Dot apparatus was then attached to a vacuum manifold and a third and final PBS wash carried out under suction.

The apparatus was disassembled, the membrane removed and quickly cut as required before being placed in blocking buffer overnight at 4oC. Membranes prepared for decorin analysis were blocked with 3% (w/v) BSA/0.1% (v/v) Tween 20 in PBS, whilst those for procollagen-I analysis were blocked with 5% (w/v) non fat dried milk powder/ 0.5% Tween 20 in PBS.

The following day, the membranes were probed with 1:10000 dilution of primary antibodies to either human procollagen-I (MAB1912; rat monoclonal; Chemicon Int. Inc., Temecula, CA) or human decorin (rabbit polyclonal; Biogenesis) for 2 hours at room temperature. The membranes were subsequently washed with TBS/ 0.05% Tween 20 (3 x 5 minutes) and then incubated with 1:1000 dilution of 125 I-conjugated anti-rat or anti-rabbit F(ab')2 fragments (Amersham) as required for 1 hour at room temperature. Following this the lmmobilon strips were again washed with TBS/Tween 20 (3 x 5 minutes) before being allowed to dry in air at room temperature.

The dried membranes were wrapped in cellophane and exposed to a Molecular Dynamics storage phosphor screen for 16-18 hours. At the end of this time the exposed screen was scanned by a phosphorimager (Molecular Dynamics Phosphorimager SF) using ImageQuant™ software. Dot intensity was assessed by computer-assisted image analysis using the quantification tools in ImageQuant™, standardised to cell number and the effects of various test reagents on decorin and procollagen-I synthesis were determined relative to a vehicle treated control value of 100 arbitrary units.

Table 1 below indicates the effects of Chromdaena Odorata extract on procollagen-I and decorin synthesis in human dermal fibroblasts, and the amounts in which it was applied. In order to normalise the results the effects of the test substance was determined relative to a vehicle treated control value of 100 arbitrary units. For comparison, a trial was performed with retinoic acid to assess its effect on decorin synthesis in human dermal fibroblasts. The concentrations of reagents used in the trials had no influence on cell viability.

The extract of Chromolaena Odorata was prepared by concentrating the aqueous extract obtained from boiling Siam weed leaves in water for 30 minutes.

The extract was tested at levels of 0.1 to 1 µg/ml in an in vitro fibroblast model containing 4% oxygen, as described in above.

The model was analysed for both procollagen-I and decorin. Their results are shown below.

**Table 1**

| **Treatment** | Procollagen I | Decorin |
|---|---|---|
| Control | 100 | 100 |
| Extract of Chromolaena Odorata at: | | |
| 0.01µg/ml | - | 172 |
| 0.1µg/ml | 113 | 180 |
| 1.0µg/ml | 116 | 182 |

The results indicate that decorin synthesis in the model is substantially up regulated by even relatively low levels (e.g. 0.01 µg/ml) of extract. Small but positive effects were also seen on procollagen I synthesis, as compared to the control. The 1.0 µg/ml sample was also comparatively tested with the retinoic acid (1µm), showing an upregulation of decorin, 138 ± 14.0 (p = 0.035, n = 4), as determined relative to a vehicle treated control value of 100 arbitrary units. This further indicates the magnitude of the increase of decorin synthesis in human dermal fibroplasts effected by Chromolaena Odorata extract, compared to the effect of retinoic acid.

### Example 2

### Fibroblast PGE2 Assay

The following example demonstrates that an extract of chromolaena odorata can effectively reduce the basal levels of prostaglandin E2 (PGE2) secreted by fibroblasts in vitro.

Primary human foreskin fibroblasts at passage 2 (P2) were seeded into 96-well plates at 10000 cells/well and maintained for 24 hours in an atmosphere of 5% carbon dioxide in Dulbeccos Modified Easgles Medium (DMEM) supplemented with 10% foetal calf serum. Chromolaena Odorata extract dissolved in ethanol was added to fresh cell media (final concentration 1% EtOH in DMEM, supplemented with 10% foetal calf serum) and added to the cells in the wells in triplicate and incubated for a further 24 hours. Phorbal myristate acetate (PMA) (Sigma) was added to the media and the cells incubated for a further 24 hours. PMA acts as the stressor which induces oxidative stress and inflammatory responses in cells. The fibroblasts/media were then analysed as described below immediately or snap frozen in liquid nitrogen and stored at -70°C for future analysis.

### Prostaglandin E2 (PGE2) assay

Volumes of 50 µl culture medium were taken for PGE2 assay after gently shaking the culture plate. PGE2 levels in the medium were determined with a Biotrak PGE2 immunoassay kit (Amersham, UK). The assay is based on the competition between unlabelled PGE2 in the sample and a fixed quantity of horseradish peroxidase labelled PGE2 for a limited amount of fixed PGE2 specific antibody. Concentrations of unlabelled sample PGE 2 are determined according to a standard curve, which was obtained at the same time.

The anti-inflammatory potential of the test compounds were assessed by the ability of the compounds to reduce the basal levels of secreted PGE2 as compared to the control. The results that were obtained are summarised in the table below and shown in Figure 1.

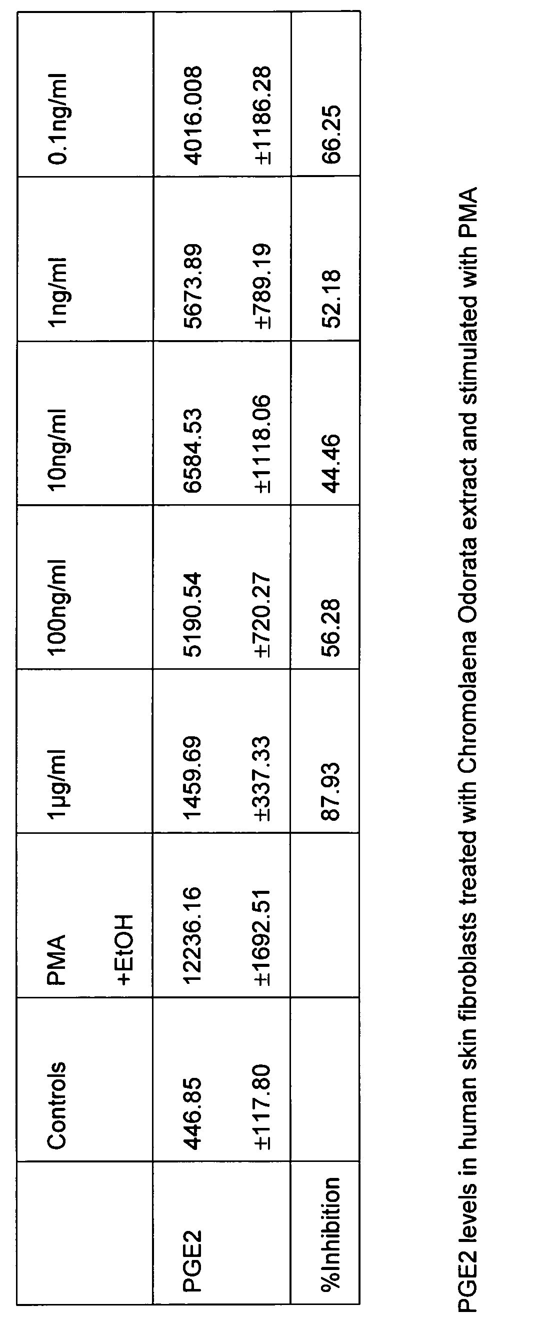

PGE2 is a well known mediator of inflammation in the skin see Greaves et al "Prostagludus, leukotriemes, phospholipase, platelet actuating factor and cytokines: an integrated approach to inflammation of human skin", Arch. Dermatol. Res. (1988) 280 [supp]: 533-541. The results indicated that fibroblasts treated with an extract of Chromolaena Odorata produce less of the pro-inflammatory prostaglandin PFE2 thereby reducing the inflammatory potential of the skin.

### Example 3

### Organ Culture - TGase Methodology

### Materials

Dettol (Reckitt & Coleman, Hull, UK), DMEM containing glutamax (Dulbecco' Modified Eagle's Medium), ABAM, (antibiotic/antimycotic X100) solution (50,000units penicillin G, 50,00µg streptomycin sulphate and 125µg amphotericin B/5mL (Gibco BRL, Paisley, UK)), hydrocortisone 21-hemisuccinate (sodium salt) (Sigma Co., Poole, UK), 35x10mm petri dishes (Nunclon, supplied by Fisher Scientific, Loughborough, UK), mini Marbrook plastic tripods (supplied by C.A. Hendley Ltd, UK), Nybolt nylon mesh (John Staniar Ltd, UK), sterile disposable 4mm biopsy punches, Steifel Labs Ltd, Bucks, UK).

Biotinylated monodansylcadaverine, Alexa Fluor 488 streptavidin, Prolong antifade solution (Molecular Probes, supplied by Cambridge Bioscience, UK), Albumin, bovine (BSA), calcium chloride CaC12, Tris hydroxymethyl aminomethane (TRIZMA base), ethylenediaminetetraacetic acid (EDTA), propidium iodide, (Sigma Co., Poole, UK) hydrochloric acid (HC1), Merck, Poole, Dorset, UK)).

### Organ Culture Method

Porcine back skin was prepared by carefully removing subcutaneous fat and muscle resulting in a skin sample 3-4mm thick. Hair was clipped and the skin was washed in water, removing any residual blood or dirt. The skin sample was cut into 7cm x 7cm squares and incubated in 5% Dettol (sterile) for 1 hr at room temp (RT). It was then incubated in DMEM containing ABAM (5mU500mL of medium) for 1hr. Hydrocortisone 21-hemisuccinate (100µg/mL, sterile filtered) was added to DMEM + ABAM. Tripods and meshes were set up on petri dishes containing 3ml of media (DMEM/ABAM/hydrocortisone), creating a negative meniscus. Punch biopsies were taken from the skin and placed onto the mesh (4 per dish), and then incubated for 24 hrs at 370C, 5% CO2 before being treated. Media was changed daily.

### In situ TGase method

A stock solution of 10mM Biotinylated monodansylcadaverine in 0.013M HC1 was, and stored at -200C. From the stock solution substrate buffer was prepared: 0.1 mM biotinylated monodansylcadaverine, 5mM CaC2 in 100mM Tris HC1. 5mM CaC12 was replaced with 200mM EDTA for a positive control.

Pig skin biopsies were snap frozen in liquid nitrogen and stored at -200C. Sections were cut (5-8 microns thick, Brights cryostat) and air dried for 10 minutes at room temperature (RT). The sections were incubated for 30 minutes at RT with 1% BSA in 0.1 M Tris/HC1, pH8.4 then incubated in substrate buffer for 2 hours at RT. The slides were washed to stop the reaction in 25mM EDTA in PBS for 5 minutes, followed by two washes in PBS. The slides were then incubated in Alexa fluor 488 streptavidin (1:250) for 30 minutes at RT, then washed in PBS as before. The slides were counterstained with 10Tg/ml propidium iodide for 10 seconds, then washed in water and mounted in prolong antifade solution. Sections were visualised using a Microscope (Leica, Leitz DMRB, Leitz ploemopak filter block L3, Milton Keynes, Bucks. UK) and images were captured using Image Grabber PC Software (Neotech Ltd. Eastleigh, Hampshire, UK).

Pig Organ Cultures were treated as described above with various concentrations of Chromolaena odorata extract for a period of 48 hours. The TGase activity in cyrostat sections of the pig organ biopsies was assayed as described and the results from the TGase assay are shown in table 3 below.

**Table 3 Effect of Chromolaena odorata extract on TGase activity in Pig organ culture at 48 hrs.**

| Treatment | | Relative expression to control |
|---|---|---|
| Control | | 100 |
| Chromolaena odorata extract | 10µg/ml | 150 |
| | 50Tg/ml | 130 |

The results in Table 3 show that there is a significant increase in TGase activity in pig skin treated with extracts of Chromolaena odorata compared to the control. Given the similarity of pig to human skin it is very likely that extracts of Chromolaena odorata would have a similar effect on TGase activity in human skin.

The data demonstrates that Chromolaena odorata extracts stimulates key enzymes in epidermal differentiation and barrier formation and thus this plant extract is of benefit to skin condition.

### Example 5

The formulation below describes an oil in water cream suitable for the methods and uses according to the present invention. The percentages indicated are by weight of the composition.

| | wt% |
|---|---|
| Mineral Oil | 4 |
| Chromolaena Odorata extract | 1.15 |
| Brij 56* | 4 |
| Alfol 16RD** | 4 |
| Triethanolamine | 0.75 |
| Butane-1,3-diol | 3 |
| Xanthan gum | 0.3 |
| Perfume | qs |
| Butylated hydroxy toluene | 0.01 |
| Water | to 100 |

| | |
|---|---|
| *Brij 56 is cetyl alcohol POE (10) | |
| ** Alfol 16RD is cetyl alcohol | |

### Example 6

The formulation below describes an emulsion cream according to the present invention.

| FULL CHEMICAL NAME OR CTFA NAME | TRADE NAME | WT. % |
|---|---|---|
| Chromolaena Odorata extract | | 2.0 |
| Disodium EDTA | Sequesterene Na2 | 0.05 |
| Magnesium aluminium silicate | Veegum Ultra | 0.6 |
| Methyl paraben | Methyl Paraben | 0.15 |
| Simethicone | DC Antifoam Emulsion | 0.01 |
| Butylene glycol 1,3 | Butylene Glycol 1,3 | 3.0 |
| Hydroxyethylcellulose | Natrosol 250HHR | 0.5 |
| Glycerine, USP | Glycerine USP | 2.0 |
| Xanthan gum | Keltrol 1000 | 0.2 |
| Triethanolamine | Triethanolamine (99%) | 1.2 |
| Stearic acid | Pristerene 4911 | 3.0 |
| Propyl paraben NF | Propylparaben NF | 0.1 |
| Glyceryl hydrostearate | Naturechem GMHS | 1.5 |
| Stearyl alcohol | Lanette 18 DEO | 1.5 |
| Isostearyl palmitate | Protachem ISP | 6.0 |
| C12-15 alcohols octanoate | Hetester FAO | 3.0 |
| Dimethicone | Silicone Fluid 200 (50cts) | 1.0 |
| Cholesterol NF | Cholesterol NF | 0.5 |
| Sorbitan stearate | Sorbitan Stearate | 1.0 |
| Butylated hydroxytoluene | Embanox BHT | 0.05 |
| Tocopheryl acetate | Vitamin E Acetate | 0.1 |
| PEG-100 stearate | Myrj 59 | 2.0 |
| Sodium stearoyl lactylate | Pationic SSL | 0.5 |
| Hydroxycaprylic acid | Hydroxycaprylic Acid | 0.1 |
| Retinyl palmitate | Vitamin A Palmitate | 0.06 |
| Alpha-bisabolol | Alpha-bisabolol | 0.2 |
| Water, DI | | q.s. to 100 |

Both the above topical compositions of example 5 and 6 provide an effective cosmetic treatment to improve the appearance of wrinkled, aged, photo-damaged, and/or irritated skin, when applied to skin that has deteriorated through the aging or photoageing or when applied to youthful skin to help prevent or delay such deteriorative changes. The compositions can be processed in conventional manner.

## Claims

1. A cosmetic method of providing at least one skin care benefit selected from treating or preventing wrinkling, sagging, aged and/or photodamaged skin; boosting decorin production in skin, boosting transglutaminase type I and type III activity in skin, increasing stratum corneum flexibility, improving skin desquamation and epidermal differentiation, lightening skin colour, controlling sebum secretion, soothing irritated, red and/or sensitive skin, improving skin texture, smoothness, firmness and generally increasing the quality of skin comprising an effective amount of an aqueous extract of Chromolaena Odorata.

2. A cosmetic method of providing at least one skin care benefit selected from treating or preventing wrinkling, sagging, aged and/or photodamaged skin; boosting decorin production in skin, boosting transglutaminase type I and type III activity in skin, increasing stratum corneum flexibility, improving skin desquamation and epidermal differentiation, lightening skin colour, controlling sebum secretion, soothing irritated, red and/or sensitive skin, improving skin texture, smoothness, firmness and generally increasing the quality of skin comprising an effective amount of an aqueous extract Chromolaena Odorata.

3. A method according to claim 2, wherein the extract of Chromolaena Odorata is an aqueous extract.

4. A method according to claim 2 or claim 3 wherein the extract is present at a level of 0.0001% to 1% by weight of the composition.

5. Use of an extract of Chromolaena Odorata for the manufacture of a topical cosmetic composition for providing at least one skin care benefit selected from Streating or preventing wrinkling, sagging, aged and/or photodamaged skin; boosting decorin production in skin, boosting transglutaminase type I and type III activity in skin, increasing stratum corneum flexibility, improving skin desquamation and epidermal differentiation, lightening skin colour, controlling sebum secretion, soothing irritated, red and/or sensitive skin, improving skin texture, smoothness, firmness and generally increasing the quality of skin.

## Amended claims

### Amended claims in accordance with Rule 86(2) EPC.

**1.** Use of an effective amount of an aqueous extract of Chromolaena Odorata for manufacture of a medicament for soothing irritated, red and/or sensitive skin.
